# EUROPEAN PATENT SPECIFICATION

(11) **EP 4 393 462 B1**
(45) Date of publication and mention of the grant of the patent: **27.08.2025**
(21) Application number: 22859917.1
(22) Date of filing: 06.05.2022
(51) Int. Cl.: A61F 13/42, G01N 27/22, A61F 13/496

(54) **INTELLIGENT ABSORBENT ARTICLE BASED ON SENSING FILM AND BUILT-IN POCKET AND RELATED DEVICE**
INTELLIGENTER SAUGFÄHIGER ARTIKEL AUF BASIS EINES SENSORFILMS UND EINER EINGEBAUTEN TASCHE SOWIE ZUGEHÖRIGE VORRICHTUNG
ARTICLE ABSORBANT INTELLIGENT BASÉ SUR UN FILM DE DÉTECTION ET POCHE INTÉGRÉE ET DISPOSITIF ASSOCIÉ

(30) Priority: 27.08.2021 CN 202110992486
(43) Date of publication of application: 03.07.2024
(73) Proprietor: Shenzhen Ediaper Technology Limited, Guangdong Province 518000 (CN); Sanwa Corporation, Tokyo 1050 004 (JP)
(72) Inventor: CHEN, Zhen, Hong Kong (CN); XU, Fei, Hong Kong (CN); WONG, Sun Hoi, Hong Kong (CN)
(74) Representative: Gulde & Partner
(86) International application number: PCT/CN2022/091190
(87) International publication number: WO 2023/024567

(56) References cited:
- EP-A1- 1 063 624
- WO-A1-2018/229017
- WO-A1-2021/078124
- WO-A1-2021/088761
- WO-A1-2021/088761
- WO-A1-99/63497
- CN-A- 1 226 855
- CN-A- 107 174 408
- CN-A- 113 425 508
- CN-A- 113 433 176
- CN-U- 205 964 299
- CN-U- 213 250 435
- US-A1- 2013 324 955
- US-A1- 2021 077 312
- US-B1- 6 246 330

## Description

### BACKGROUND

### Priority Claim

This disclosure claims the priority of patent application No. 202110992486.1 filed with the Chinese Patent Office on August 27, 2021, entitled "A smart absorbent article with sensing lines and an invisible pocket", all of which are in the present disclosure.

### Technical Field

The present disclosure relates to an absorbent article, and in particular, to a smart disposable absorbent article and a related device based on a capacitive sensing film and a built-in pocket.

### Related Art

Disposable absorbent articles include diapers, nappies, sanitary napkins, urine pads and other sanitary products, which are disposable care products and should be replaced as needed when in use. If changed too early/ too often, it will increase workload and cause waste; If changed too late, it is easy to cause leakage, and the excrement stimulates the skin for a long time and is easy to cause skin diseases. Therefore, people look forward to a smart disposable absorbent article that can detect moisture/wetness in real time and prompt to change the diaper at the right time, which has a positive significance for scientific use of the products. Smart absorbent articles are known from WO2018/229017A1 and WO2021/088761A1.

In terms of prior art, China Patent publication No. CN107174408B discloses an absorbent article and related method, which realizes the accurate, reliable, and quantified wetness detection function based on electrolytic capacitance by setting two sensing lines (flexible electrodes) on the outer surface of the backsheet of the diaper, which is one of the most representative solutions among the prior art. When it is used, a sensor is attached to the outer surface of the diaper first, and then open the sensor flap, align the probe with the sensing lines on the diaper, and close it, so that the probe can get connected with the sensing lines and detect the wetness state of the diaper in real time.

The shortcomings of the above prior art mainly include: (1) the sensing lines are printed on the outer surface of the backsheet of the diaper with carbon ink, which will produce two black lines and affect the appearance of the diaper. (2) the sensing lines set on the outer surface of the backsheet of the diaper is easy to be affected by external moisture, such as touching the diaper with a wet hand will cause false alarm. (3) the sensor is hard to secure on the outside of diaper. (4) the sensor probe must be tied through the outer non-woven fabric of the diaper to connect with the sensing lines, which is easily lead to poor contact. (5) the alignment between the sensor and the sensing lines depends on the naked eye and experience, which is easy to cause misalignment and connection failure.

In order to solve the above problems, China Patent publication No. CN1226855A discloses a wetness detection device for diapers and other diapers with such a device. Its sensing lines are arranged inside the diaper and a pocket is arranged on the outer surface of the diaper, and the elasticity of the pocket itself holds the sensor in place, and the sensor located on the outside of the diaper is then capacitively coupled to the sensing lines located inside the diaper for wetness detection.

The main shortcomings of this prior art include: (1) the sensor and the sensing lines are not face-to-face contact; it is difficult to achieve a reliable electrical connection. (2) the pocket and the sensing lines are located on both sides of the diaper, easy to cause dislocation. (3) the sensing lines detect wetness by direct contact with urine, which is a traditional resistive type of wet alarm system and cannot achieve accurate and reliable wetness level detection based on electrolytic capacitance. (4) the pocket set outside the diaper cannot use the waist elasticity generated by the diaper in the wearing state to fix the sensor and cannot make the electrical connection between the sensor contacts and the sensing lines more reliable.

The shortcomings of the prior art need to be solved by new technical solutions.

### SUMMARY

The technical problems to be solved by the present disclosure are mainly the deficiencies in the prior art mentioned in the background. In order to solve these problems, the measures taken by the present disclosure mainly include:
1. The sensing lines will be set inside the diaper to solve the problem that the sensing lines affects the appearance of the diaper and is affected by external moisture.
2. Set up a short-circuit protection mechanism in the diaper, so that the sensing lines are protected from the influence of moisture inside the diaper, and achieve accurate, reliable, and quantifiable wetness detection function based on electrolytic capacitance.
3. Set a pocket inside the diaper to solve the problem of fixing the sensor.
4. Make the sensor contacts face to face with the sensing lines to solve the reliability of the electrical connection.
5. Take measures to make the built-in pocket can be automatically centered and aligned with the sensing lines to ensure that the sensor will not be misaligned when inserted into the pocket.
6. Provide a sensing film with a built-in pocket as a raw material for the production of smart diapers, which brings convenience to the mass production of products.
7. Provide a solution that can use the elastic force generated on the waist when the diaper is worn to fix the sensor and make the electrical connection between the sensor and the sensing lines more reliable.

In order to solve the above technical problems, in the first aspect, the present disclosure provides a smart absorbent article as defined by the appended set of claims.

At least part of the sensing lines is located within the contact surface and includes exposed part, the short-circuit protection mechanism includes a waterproof covering layer which covers the contact surface, and a separable part between the contact surface and the waterproof covering layer constitutes the built-in pocket, the sensing lines can realize the wetness detection function of the absorbent article through the dielectric layer by means of electrolytic capacitance.

The capacitive sensing film includes a sensing film with a head-and-tail built-in pocket, the sensing film includes a plastic film substrate, the sensing lines are arranged on one surface of the plastic film substrate, the waterproof covering layer is covered on the sensing lines, the waterproof covering layer and the plastic film substrate include a mutually bonded part in the middle section and constitute a sensing area, and include a mutually separable part at both ends of the head and tail and constitute the built-in pocket, the outer surface of the plastic film substrate or the waterproof covering layer is oriented towards the absorbent layer; or

The capacitive sensing film includes a sensing film with a through-type built-in pocket, the sensing film includes a plastic film substrate, the sensing lines are arranged on one surface of the plastic film substrate and constitutes the contact surface, the other surface of the plastic film substrate constitutes the sensing surface, the plastic film substrate constitutes the dielectric layer, the waterproof covering layer is covered on the contact surface, the edge of both sides of the waterproof covering layer and the plastic film substrate includes a part bonded to each other and constitutes a boundary preventing liquid penetration into, a hollow part is further included between the waterproof covering layer and the plastic film substrate and constitutes the through-type built-in pocket.

Wherein the disposable absorbent article includes a diaper, an insertion pad, a sanitary napkin, a maternal towel or an urine pad, the topsheet includes a hydrophilic non-woven fabric, the absorbent layer includes polymer absorbent material, the backsheet includes a breathable or impermeable PE film, the sensing lines include ink lines printed with conductive ink, metal lines formed by physical vapor deposition, aluminum foil or copper foil, the plastic film substrate include a PE, PP, CPP, BOPP or PET film, and the waterproof covering layer includes a plastic film, a waterproof paper or a water-repellent non-woven fabric, the waterproof protective layer includes a polymer waterproof coating or a polymer waterproof composite layer, and the electrolyte containing liquid includes urine, loose stool, sweat or blood containing salt.

Wherein when the disposable absorbent article is soaked by the liquid containing electrolytes, the sensing lines, the dielectric layer, and the liquid together to form an electrolytic capacitor, where the sensing lines constitute the electrodes of the electrolytic capacitor, the dielectric layer constitutes the dielectric medium of the electrolytic capacitor, the liquid constitutes the electrolyte of the electrolytic capacitor, the wetness level of the disposable absorbent article is proportional to the capacitance of the electrolytic capacitor, the capacitance is proportional to the area of the liquid corresponding to the sensing lines, is proportional to the dielectric constant of the dielectric layer, and is inversely proportional to the thickness of the dielectric layer.

In the second aspect, the present disclosure provides a capacitive sensing film as defined by the appended set of claims.

Wherein the sensing lines include ink lines printed by conductive ink, metal lines formed by physical vapor deposition, aluminum foil or copper foil, and the plastic film substrate includes a PE, PP, CPP, BOPP or PET film, the waterproof covering layer includes a plastic film, a waterproof paper, a water-repellent non-woven fabric or a hot melt adhesive film.

Wherein the sensing lines include ink lines printed by conductive ink, metal lines formed by physical vapor deposition, aluminum foil or copper foil, and the plastic film substrate includes a PE, PP, CPP, BOPP or PET film, the waterproof covering layer includes a plastic film, a waterproof paper, a water-repellent non-woven fabric or a hot melt adhesive film.

In the fourth aspect, the present disclosure provides a system device for monitoring the wet state of a smart absorbent article as defined by the appended set of claims.

Wherein the sensor includes at least three contacts, two of which constitute an insertion detection device, when the sensor is inserted into the built-in pocket and the two contacts contact the same sensing line at the same time, the insertion detection device is triggered, and the sensor enters working state.

Wherein the sensor also includes a wireless transmitter and a wireless receiving device for sending and receiving wetness information of the smart absorbent article by wireless means, and the wireless receiving device includes an audible and visual alarm device, a smart phone, or a personal computer.

The present disclosure has the beneficial effect of solving the shortcomings of the prior art mentioned in the background, including: (1) the sensing lines are successfully arranged in the inside of the diaper, eliminating the influence of the color of the sensing lines on the appearance of the diaper. (2) the sensing lines are protected from short circuit and are no longer affected by moisture inside or outside the diaper. (3) the pocket set inside the diaper can more effectively contain and fix the sensor. (4) the sensor and the sensing lines to achieve face to face contact and make the connection more reliable. (5) the built-in pocket and sensing film are automatically positioned and aligned to avoid misalignment. (6) the sensing film can be used as a general production material, with a wider range of versatility and convenience. (7) the elastic force generated on the waist when the diaper is worn is cleverly used to fix the sensor and make the connection between the sensor and the sensing lines more reliable.

The present disclosure solution effectively improves the reliability and convenience of the product and creates favorable conditions for the development of the diaper industry and smart upgrade of diaper products.

### BRIEF DESCRIPTION OF THE DRAWINGS

In order to state the technical scheme of the embodiments of the present disclosure more clearly, the following is a brief introduction of the drawings required to be used in the embodiments. Obviously, the drawings described below are only some embodiments of the preset disclosure, and other drawings can be obtained according to these drawings for ordinary technicians in the field without creative effort.
FIG. 1 illustrates a schematic diagram of the appearance structure of a smart absorbent article when it is used together with a sensor according to an embodiment of the present disclosure.
FIG. 2A and FIG. 2B illustrate a three-dimensional structure diagram and a cross section structure diagram/equivalent circuit diagram of a single-sided sensing film suitable for the smart absorbent article according to an embodiment of the present disclosure.
FIG. 3A and FIG. 3B illustrate a front structure diagram and a D-D' cross section structure diagram of a capacitive sensing film including a head-and-tail built-in pocket suitable for used in a smart absorbent article according to an embodiment of the present disclosure.
FIG. 4A, FIG. 4B and FIG. 4C illustrate a front structure diagram, an E-E' cross section structure diagram and a position relationship diagram of a capacitive sensing film with a through-type built-in pocket, which are suitable for the use of a smart absorbent article according to an embodiment of the present disclosure.
FIG. 5 illustrates a schematic diagram of the appearance structure of a smart absorbent article (with a built-in pocket on the elastic waist) according to an embodiment of the present disclosure.
FIG. 6 illustrates a schematic diagram of the appearance structure of a smart absorbent article according to an embodiment of the present disclosure, whose outer face includes an incision leading to a built-in pocket.
FIG. 7 and FIG. 8 illustrate schematic diagrams of appearance structure of sensing films suitable for use in a smart absorbent article having a curved sensing lines design and a plurality of sensing lines according to an embodiment of the present disclosure.

### DETAILED DESCRIPTION

The descriptions of the following embodiments refer to the attached drawings and serve as examples of specific embodiments in which the present disclosure can be implemented. The direction and position terms mentioned in the present disclosure, such as "up", "down", "front", "back", "left", "right", "inside", "outside", "top", "bottom", "side", etc., only refer to the direction or position of the attached drawings. Therefore, the direction and position terms used are used to describe and understand the present disclosure, and not to limit the scope of protection of the present disclosure.

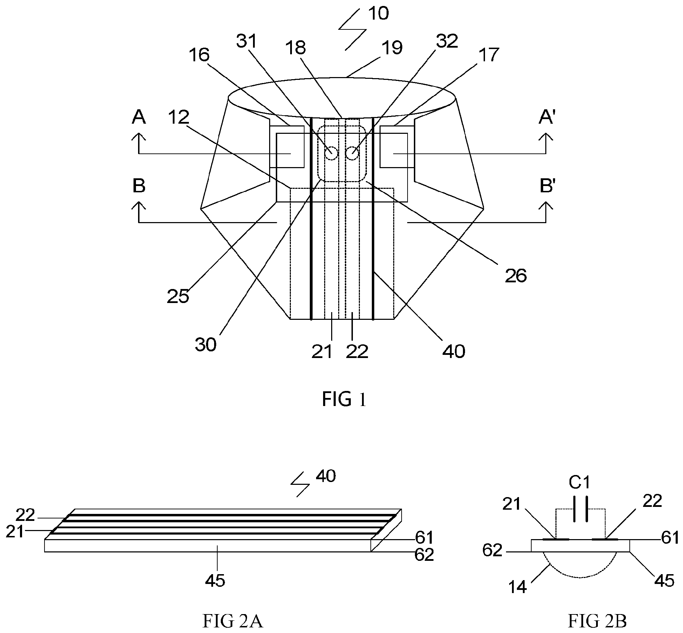

FIG. 1 is a schematic diagram of the appearance structure of a smart absorbent article when it is used together with a sensor in an embodiment of the present disclosure, which is almost the same in appearance as a traditional disposable absorbent article. Diapers are the most representative of disposable absorbent articles, and the embodiment of the present disclosure will mainly take diapers as an example to illustrate, and the relevant descriptions are also suitable for disposable absorbent articles such as pull-up pants, training pants, insertion pads, sanitary napkins, maternity napkins, urine pads, etc. In most cases, the diapers discussed in embodiments of the present disclosure have the same meaning as disposable absorbent articles.

The smart absorbent article10 of the embodiment of the present disclosure is realized on the basis of a traditional diaper by adding some materials or devices that can sense (detect) wetness (moisture), which make the diaper smart, so it can be referred to as a smart diaper. Diapers usually include a backsheet (leakproof layer), an absorbent layer (sandwich), a topsheet (dry layer), an outer non-woven fabric (composite layer) and other components, the backsheet usually includes a breathable or non-breathable polyethylene film (PE), the absorbent layer usually includes polymer absorbent material (SAP) and wood pulp, the topsheet usually includes a soft and comfortable hydrophilic non-woven fabric. The outer layer of non-woven fabric is usually combined with the backsheet to form a composite backsheet, which can make the backsheet have a better feel and increase the strength of the backsheet.

The front 18 of the diaper can also be referred to as the front belly, front waist, or diaper head, while the back 19 of the diaper can be referred to as the rear, back waist, or diaper tail. A typical diaper may also include left and right stickers/ adhesive strips/ Velcro 16 and 17, which are located on the left and right sides of the front abdomen of the diaper respectively and are attached to the front belly patch /front waist patch 25 when in use, which pulls the waist of the diaper tight to prevent the diaper from slipping off.

In terms of wetness detection, the embodiment of the present disclosure is mainly realized by setting a capacitive sensing film (referred to as sensing film) material containing detection electrodes inside the diaper. The sensing film includes at least two detection electrodes/ flexible electrodes/ wetness sensing lines (collectively referred to as sensing lines). For example, the sensing film 40 in this embodiment includes a first sensing line 21 and a second sensing line 22. The sensing lines usually start at the front edge of the diaper and reach or approach the back edge of the diaper across the absorbent layer 12 area (crotch) of the diaper to achieve wetness detection of the entire diaper. The smart absorbent article of the embodiments of the present disclosure also includes a built-in pocket 26 below the front belly patch 25 to accommodate a sensor 30, and the sensing film/ sensing lines is at least partially located within the pocket to contact and perform electrical connection with the sensor inserted into the pocket.

The sensing film 40 and the built-in pocket 26 can be arranged between the backsheet and the topsheet of the diaper or between the backsheet and the outer non-woven fabric. This embodiment uses the gap in the interlayer at the front edge of the diaper as an opening for the built-in pocket 26 to insert the sensor. The sensor 30 in this figure includes two contacts 31 and 32, which are in face-to-face contact with the first and second exposed sensing lines 21 and 22 in the built-in pocket and electrically connected and can detect the wetness by means of electrolytic capacitance, thus realizing the wetness detection function of the diaper.

The sensing film of the embodiments of the present disclosure is a flexible film including two outer surfaces, and the surface in contact with the excrement/liquid to be measured is called the sensing surface. The sensing film can also be divided into contact area and sensing area, the sensing area is usually corresponding to the absorbent layer of the diaper and is mainly used to detect the wetness of the diaper, while the contact area is mainly used to contact the sensor contacts and implement electrical connection. The plane in contact with the sensor contacts is called the contact surface, and at least part of the contact surface is located within the built-in pocket 26. And at least part of the sensing lines is exposed on the contact surface in order to implement electrical connection with the contact of the sensor.

The sensing lines of the embodiments of the present disclosure is non-contact with the liquid to be measured, and the sensing lines will implement the wetness detection of the diaper through non-contact capacitive sensing (capacitive coupling). In order to prevent the liquid from penetrating into the sensing lines area, the embodiments of the present disclosure also include a short-circuit protection mechanism, which is to prevent the sensing lines from coming into contact with the liquid containing electrolytes to short-circuit and cause detection failure or lead to false triggering/ false alarm. Because the sensing film is arranged inside the diaper, the diaper of the embodiment of the present disclosure is no different from the traditional diaper from the outside, and the sensing film/ sensing line has almost no influence on the appearance of the diaper. In order to illustrate the relationship between the sensing film, sensing line, contact surface, sensing surface of the embodiment of the present disclosure and the topsheet, absorbent layer, backsheet and outer non-woven fabric of the diaper, an A-A' and a B-B' sections related to the embodiment shown in Figure 1 are further explained in the following legends.

FIG. 2A and FIG. 2B are three-dimensional structure diagram and cross section structure diagram/equivalent circuit diagram of a single-sided sensing film suitable for a smart absorbent article of an embodiment of the present disclosure. The sensing film 40 of the present embodiment is a single-sided capacitive sensing film including a film substrate (the substrate) 45 and a first sensing line 21 and a second sensing line 22 arranged on one surface of the substrate. The sensing lines will detect the wetness state on the other surface of the substrate 45 by means of electrolytic capacitance.

The sensing lines in the embodiments of the present disclosure generally include ink lines (conductive film) printed with conductive ink (such as carbon paddle or silver paste), metal lines (metal film) of physical vapor deposition (such as vacuum evaporation aluminum) or aluminum foil, copper foil, etc. In this embodiment, the surface of the substrate 45 with sensing lines is referred to as the contact surface 61, and the other surface as the sensing surface 62, and the substrate 45 insulating the sensing lines and the sensing surface separated from each other as the dielectric layer. Commonly used substrates include PE (polyethylene) film, PP (polypropylene) film, CPP (casting polypropylene) film, BOPP (biaxial tensile polypropylene) film, PET (polyester) film and other plastic films, so it can be called plastic film substrate, its thickness is generally between 5 microns and 50 microns.

When a liquid containing electrolyte (such as urine containing salt, loose stool, sweat, menstrual blood, blood and other liquids, referred to as liquid) 14 accumulates on the sensing surface 62, the sensing lines 21 and 22 together with the liquid 14 and the plastic film substrate 45 form an electrolytic capacitor C1, in which the sensing lines 21 and 22 form the electrodes of C1. The plastic film substrate 45 constitutes the dielectric of C1, the liquid 14 constitutes the electrolyte of C1, the capacitance of C1 is inversely proportional to the thickness of the dielectric, is proportional to the dielectric constant, and is proportional to the area corresponding to the liquid and the electrodes (sensing lines), by reading the capacitance value of C1 can accurately know the quantitative wetness state of the sensing surface.

It should be noted that the electrolytic capacitance of the embodiments of the present disclosure is generated based on the conductivity of human excrement, which is essentially different from the dielectric capacitor mentioned by some prior art which treats urine as a non-conductive liquid with a high dielectric constant (e.g. 80). In fact, there is also some dielectric capacitance unrelated to electrolytic capacitor generated between the first and second sensing lines of the embodiments of the present disclosure, but this dielectric capacitance is much smaller than the electrolytic capacitance when the diaper is wet, so the embodiments of the present disclosure ignore the dielectric capacitance and only discuss the electrolytic capacitance generated by the electrolyte liquid.

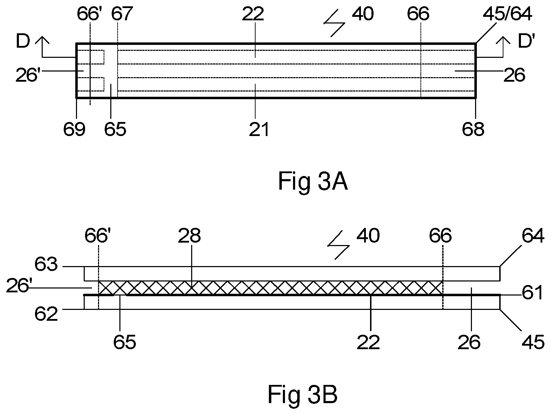

FIG. 3A and FIG. 3B are the front structure diagram and D-D' cross section structure diagram of a capacitive sensing film including a head-and-tail built-in pocket suitable for used in a smart absorbent article as an embodiment of the present disclosure. In the aforementioned embodiments, the sensing film itself has no pockets and needs to be combined with other materials, such as the backsheet 15 or the outer non-woven fabric 13, to form the built-in pocket. In present embodiment, a waterproof covering layer is integrated into the sensing film in advance to form a sensing film with its own pocket and short-circuit protection mechanism, which can facilitate the application of the sensing film and the production of the smart diapers.

The sensing film 40 of the present embodiment includes a plastic film substrate 45, sensing lines 21/ 22 arranged on one surface of the plastic film substrate, and a waterproof covering layer 64 covering the sensing lines, which fully protects the sensing lines 21 and 22. The waterproof covering layer 64 and the plastic film substrate 45 include both bonded part (at middle section) and separable part (at ends of the head and tail), wherein a separable part included at the front edge 68 constitutes a functional pocket 26, and a separable part included at the rear end edge 69 of the sensing film constitutes a redundant pocket 26'. Sensing lines 21 and 22 are exposed in the two pockets, and the plane where the sensing lines are located constitutes a contact surface 61, so as to contact and implement electrical connection with the contacts of the sensor inserted in the pocket, and the corresponding area constitutes a contact area of the sensing film.

From the bottom boundary 66 of pocket 26 to the bottom boundary 66' of pocket 26', the waterproof covering layer 64 is bonded together with the plastic film substrate 45, which forms part of the short-circuit protection mechanism and forms a sensing area of the sensing film, which can be used to detect wetness on the outer surface 62 of the plastic film substrate 45 or the outer surface 63 of the waterproof covering layer 64. A breakpoint 65 is also included in this embodiment to make the head and tail ends of the sensing lines 21 and 22 disconnected from each other to prevent the normal operation of the sensing lines in the functional pocket 26 from being affected by short circuits in the sensing lines in the redundant pocket 26'. In addition, breakpoint 65 can also be used as a positioning marker to help set the sensing film in the appropriate position of the diaper.

When the sensing film is set inside the diaper for use, the front edge 68 will usually be flush with the front edge 18 of the diaper, and the sensing area may correspond to the absorbent layer of the diaper. The embodiment may either use the plastic film substrate 45 as the dielectric layer and the outer surface 62 as the sensing surface or use the waterproof covering layer 64 as a dielectric layer and the outer surface 63 as the sensing surface. Sensing lines 21 and 22 can detect the wetness state of the sensing surface through the corresponding dielectric layer by means of electrolytic capacitance, and the detection sensitivity (the ability to generate electrolytic capacitance) is proportional to the dielectric constant of the dielectric layer and inversely proportional to the thickness of the dielectric layer.

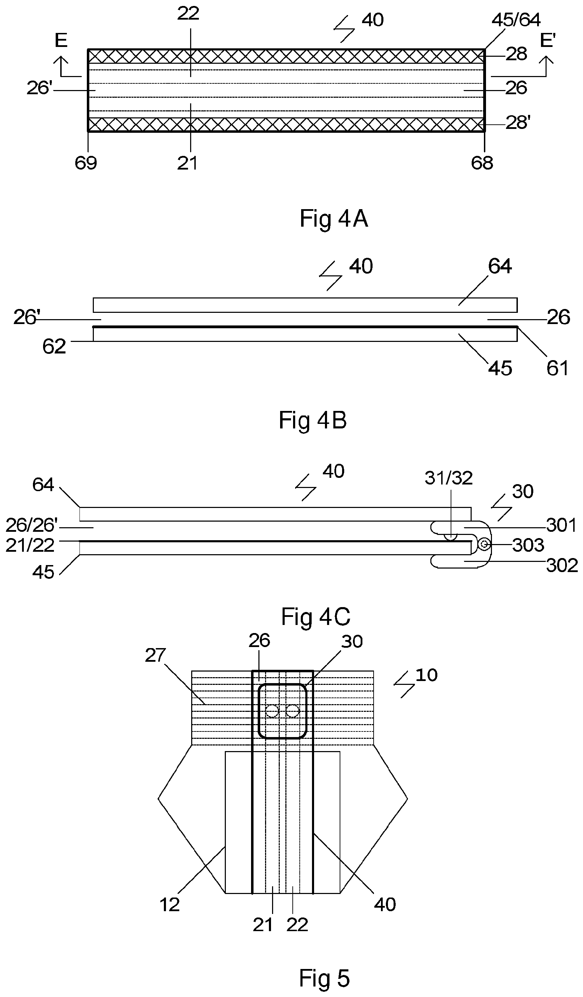

FIG. 4A, FIG. 4B and FIG. 4C are the front structure diagram, the E-E' cross section structure diagram and the position relationship diagram of a capacitive sensing film of an embodiment of the present disclosure, this kind of sensing film includes a through-type built-in pocket and is suitable for use in the smart absorbent article of the embodiment of the preset disclosure. Unlike the embodiments shown in FIG. 3A and FIG. 3B, which are bonded in the middle section of the sensing film, the plastic film substrate 45 and the waterproof covering layer 64 in this embodiment are bonded at the edges of both sides, forming bonding zones 28 and 28' respectively, which can be bonded by adhesive or hot pressing. When hot pressing bonding is selected, EVA, TPU, PES, PVC or other hot melt adhesive film can be selected as waterproof covering layer. In this embodiment, the edges of the two sides of the sensing film are bonded together to form a boundary that prevents liquid penetration and constitutes a short-circuit protection mechanism of the embodiment of the present disclosure, so that the sensing lines 21 and 22 are effectively protected, and the hollow part constitutes a through-type built-in pocket, and the through-type pocket includes a pocket with opening at the left and right ends of the sensing film respectively, which are a functional pocket 26 and a redundant pocket 26'.

In this embodiment, the surface of the plastic film substrate 45 with sensing lines 21 and 22 is the contact surface 61, and the other surface of the plastic film substrate 45 where the sensing lines does not exist is the sensing surface 62, the plastic film substrate 45 constitutes the dielectric layer, and the sensing lines 21 and 22 detect the wetness state on the sensing surface 62 through the dielectric layer 45 by means of electrolytic capacitance. The sensing film in this embodiment is not divided into a sensing area and a contact area, or the sensing film in any segment contains both the sensing area and the contact area. The sensing film in this embodiment is itself equipped with the built-in pockets and the short-circuit protection mechanism and can be easily set for use on any layer of the diaper. At the same time, because the sensing film in this embodiment is completely symmetrical, it does not need to be positioned in the production process of smart absorbent articles, and these characteristics make it more versatile and convenient in application.

The sensing film of this embodiment can be cut off at any length for use, e.g. it may be selected to be as long as the diaper so that the front edge 68 is flush with the front edge 18 of the diaper, or it may be selected to be longer than the diaper so that the front edge 68 protrudes from the front edge 18 of the diaper so that the sensor can be inserted for use from the protruding part. In order to facilitate production, the sensing film can also be packed into a large roll of coil form, each roll of sensing film can produce thousands of smart diapers, making the sensing film to be a general smart diaper production raw material.

Since the pocket of this embodiment is a through-type pocket, in order to enable the sensor to be fixed in the pocket, a clamshell-type design as shown in Figure 4C can be used. The sensor 30 in the figure includes an insert section 301 and a clamshell section 302, both of which are hinged by hinge 303. When using, you can first open the clamshell 302, and then insert the insert section 301 into the built-in pocket 26, so that contact 31/32 and sensing lines 21/22 face to face contact, and then close the clamshell 302 to clamp the plastic film substrate 45, which not only makes the contact between the sensor contact and the sensing lines more reliable, also prevents the sensor from slipping out or into deeper positions. The sensor design is applicable not only to the present embodiment, but also to other embodiments of the present disclosure.

FIG. 5 is a schematic diagram of the appearance structure of the smart absorbent article including a built-in pocket on the elastic waist of an embodiment of the present disclosure. The difference from the diapers in the other embodiments above is that the diaper in the present embodiment is a panty diaper with an elastic waistline 27, such as pull-ups, training pants, etc. The built-in pocket 26 is arranged in the interlayer of the elastic waistline 27. When the diaper is in the wearing state, the elastic force generated on the elastic waistline can be converted into the vertical force pressing the built-in pocket 26 to the user's body, which can effectively press and fix the sensor 30 in the pocket 26. The contact point of the sensor 30 and the sensing lines 21 and 22 on the contact surface of the sensing film 40 are made more reliable.

The sensing area of the sensing film 40 in this embodiment corresponds to the absorbent layer 12 of the diaper, while the contact area of the sensing film 40 is located within the built-in pocket 26 of the elastic waist of the diaper, that is, the two are separated from each other in position to further prevent the moisture in the absorbent layer 12 from penetrating into the contact area and causing short circuit of the sensing lines, which can also be regarded as a short-circuit protection mechanism. The sensing film 40 may adopt any of the above embodiments, and the built-in pocket 26 may extend to the edge of the diaper waist and include in the sandwich gap at the edge an opening to the built-in pocket through which a sensor 30 performing wetness detection can be inserted.

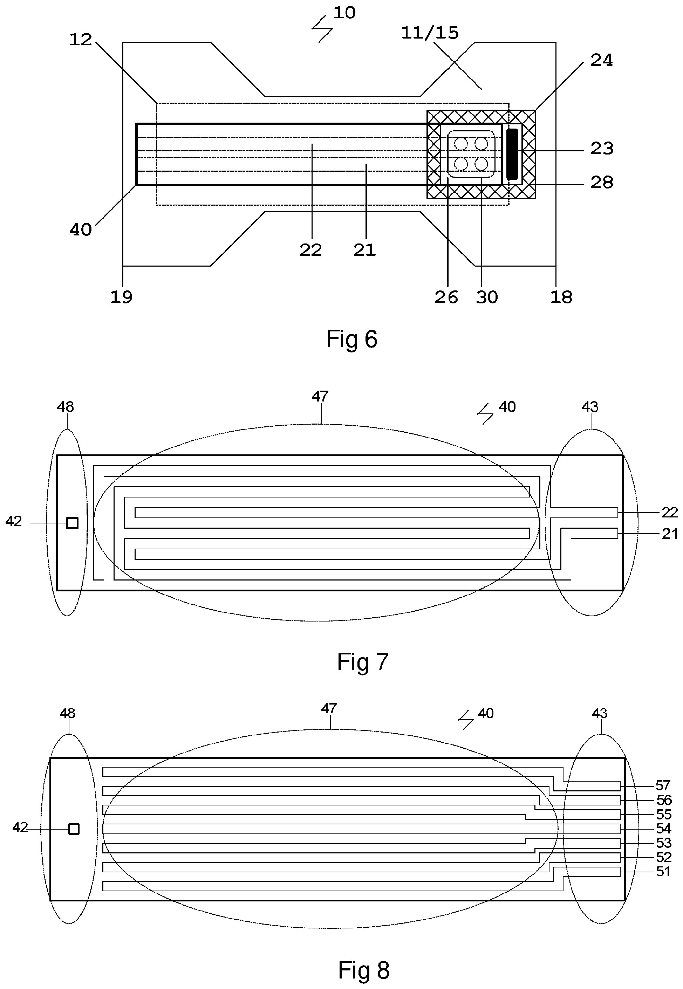

FIG. 6 is a schematic diagram of the appearance structure of a smart absorbent article as an embodiment of the present disclosure, whose outer face includes an incision leading to a built-in pocket. In the aforementioned embodiments, the opening of the functional pocket 26 is generally located at the front edge of the diaper, and the sandwich gap between the contact surface 61 of the sensing film and the backsheet 15 or the waterproof covering layer 64 is used as an invisible opening. In practical applications, the sensor can also be placed into the built-in pocket by cutting a notch on the outer or inner surface of the diaper.

The smart diaper 10 in the present embodiment includes a topsheet 11, an absorbent layer 12, a backsheet 15, a sensing film 40, a waterproof covering layer 64 and other components. The sensing film includes sensing lines 21 and 22, and the contact surface of the sensing film 40 includes a mutually separable part between the waterproof covering layer or the backsheet and constitutes a built-in pocket 26, an incision 23 is also included on the outer surface of the diaper or on the inner surface at the front edge, and the incision 23 leads to the built-in pocket 26 through which the sensor 30 can be placed into the built-in pocket 26 for use.

FIG. 7 and FIG. 8 are schematic diagrams of the appearance structure of a sensing film having a toroidal sensing lines design and a plurality of sensing lines designed for the smart absorbent article of the embodiments of the present disclosure. The sensing film 40 in the figure includes a contact area 43, a sensing area 47 and a redundant area 48. In application, the contact area 43 corresponds to the front edge of the diaper and includes bare sensing lines and constitutes a contact surface where a functional pocket can be generated for contact and electrical connection with the inserted sensor contacts; Redundancy area 48 corresponds to the back edge of the diaper, where redundant pockets are generated and redundancy is provided for the diaper cutting; The sensing area 47 corresponds to the position of the crotch of the diaper and is used to detect the wetness state of the diaper

In the embodiment shown in FIG. 7, the sensing area 47 includes a curved sensing lines design, which includes 6 sensing lines from top to bottom, in which singular sensing lines are connected with the sensing line 22, and even number of sensing lines are connected with the sensing line 21, this method can effectively expand the coverage area and detection range of sensing area 47. The sensing area 47 of the embodiment shown in FIG. 8 consists of seven parallel sensing lines (from 51 to 57), which is also designed to effectively extend the coverage of the sensing lines and provide more wetness status information at the location. It should be noted that these sensing line patterns in this embodiment are asymmetrical from end to end. In order to enable the sensing film to be accurately embedded in the specific position of the diaper, a positioning mark 42 is also included on the sensing film 40.

In addition, the sensing film of the embodiment of the present disclosure can also have more structural forms and more sensing line pattern designs, and corresponding changes can be made on the basis of the above-mentioned embodiments combined with actual needs. In addition, in the aforementioned embodiments, the built-in pocket is arranged on the front straight face of the diaper, and in practical application, it can also be arranged on other positions of the diaper, such as the crotch, back or side position of the diaper, as long as there is a sensing film/sensing lines through these places.

Because the smart absorbent article 10 of the embodiments of the present disclosure has a smart wetness detection function, a smart wetness state monitoring system can be constituted by inserting a sensor 30 into the built-in pocket 26. In such a monitoring system, the sensor will include at least two contacts for contact and electrical connection with the first and second sensing lines, and the sensor will also include a capacitance detection device to detect the wetness state of the absorbent article by means of electrolytic capacitance. In practical applications, the sensor preferably has four or more contacts, two of which can form an insertion detection device. The sensor can also usually include a wireless transmitter, and a wireless receiving device can be set up to accompany it, so that the wetness status information of the absorbent article can be sent and received wirelessly, and the wireless receiving device usually includes an audible and visual alarm device, a smart phone, or a personal computer.

The foregoing descriptions are merely exemplary embodiments of the present disclosure, and certainly are not intended to limit the scope of claims of the present disclosure. Therefore, equivalent variations made in accordance with the claims of the present disclosure shall fall within the scope of the present disclosure.

## Claims

1. A smart absorbent article (10) based on a capacitive sensing film (40) and a built-in pocket (26), comprising a disposable absorbent article, a capacitive sensing film (40), a built-in pocket (26) and a short-circuit protection mechanism, the disposable absorbent article comprises a topsheet (11), an absorbent layer (12) and a backsheet (15), and the sensing film (40) comprises a contact surface (61), a sensing surface (62), a dielectric layer and at least two sensing lines (21,22), the outer surface of the dielectric layer constitutes the sensing surface, the dielectric layer separates and insulates the sensing lines (21,22) and the sensing surface (62), when the sensing surface (62) is soaked by a liquid containing electrolyte, the sensing lines (21,22) can realize the wetness detection function of the sensing surface (62) through the dielectric layer by means of electrolytic capacitance, the built-in pocket (26) is adapted to insert a sensor, the contact surface (61) facilitates face-to-face contact and electrical connection between the sensor and the sensing lines (21,22), the short-circuit protection mechanism is adapted to protect the sensing lines (21,22) from moisture inside and outside the disposable absorbent article;
wherein at least part of the sensing lines (21,22) is located within the contact surface (61) and includes exposed part, the short-circuit protection mechanism comprises a waterproof covering layer (64) which covers the contact surface (61), and a separable part between the contact surface (61) and the waterproof covering layer (64) constitutes the built-in pocket (26), the sensing lines (21,22) can realize the wetness detection function of the absorbent article through the dielectric layer by means of electrolytic capacitance; and
the capacitive sensing film (40) comprises a sensing film with a head-and-tail built-in pocket, the sensing film comprises a plastic film substrate (45), the sensing lines (21,22) are arranged on one surface of the plastic film substrate (45), the waterproof covering layer (64) is covered on the sensing lines (21,22), the waterproof covering layer (64) and the plastic film substrate (45) include a mutually bonded part in the middle section and constitute a sensing area, and include a mutually separable part at both ends of the head and tail and constitute the built-in pocket 26, the outer surface of the plastic film substrate (45) or the waterproof covering layer (64) is oriented towards the absorbent layer (12); or
the capacitive sensing film (40) comprises a sensing film with a through-type built-in pocket, the sensing film comprises a plastic film substrate (45), the sensing lines (21,22) are arranged on one surface of the plastic film substrate (45) and constitutes the contact surface (61), the other surface of the plastic film substrate (45) constitutes the sensing surface (62), the plastic film substrate (45) constitutes the dielectric layer, the waterproof covering layer (64) is covered on the contact surface (61), the edge of both sides of the waterproof covering layer (64) and the plastic film substrate (45) includes a part bonded to each other and constitutes a boundary preventing liquid penetration into, a hollow part is further included between the waterproof covering layer (64) and the plastic film substrate (45) and constitutes the through-type built-in pocket.

2. The smart absorbent article (10) according to claim 1, wherein the disposable absorbent article comprises a diaper, an insertion pad, a sanitary napkin, a maternal towel or an urine pad, the topsheet (11) comprises a hydrophilic non-woven fabric, the absorbent layer (12) comprises polymer absorbent material, the backsheet (15) comprises a breathable or impermeable PE film, the sensing lines (21,22) comprise ink lines printed with conductive ink, metal lines formed by physical vapor deposition, aluminum foil or copper foil, the plastic film substrate comprise a PE, PP, CPP, BOPP or PET film, and the waterproof covering layer (64) comprises a plastic film, a waterproof paper or a water-repellent non-woven fabric, and the electrolyte containing liquid comprises urine, loose stool, sweat or blood containing salt.

3. A capacitive sensing film (40) including a head-and-tail built-in pocket for making the smart absorbent article (10) described in claim 1, comprising a plastic film substrate (45), a waterproof covering layer (64) and at least two sensing lines (21,22), the sensing lines (21,22) are arranged on one surface of the plastic film substrate (45), the waterproof covering layer (64) covers the sensing lines (21,22), the waterproof covering layer (64) and the plastic film substrate (45) include a mutually bonded part in the middle part to form a protected sensing area for preventing liquid penetration into the area and causing a short circuit of the sensing lines (21,22), the waterproof covering layer (64) and the plastic film substrate (45) include a mutually separable part at both ends of the head and tail and constitute a contact area, wherein the contact area located at the front edge constitutes a functional pocket, the sensing lines (21,22) are exposed in the functional pocket and constitutes a contact surface (61) for face-to-face contact and electrical connection with the contact points of the sensor inserted into the pocket, and the contact area located at the back end edge constitutes a redundant pocket, which can provide error redundancy for the positioning cutting during the production of the smart absorbent article (10); and
the plastic film substrate (45) or the waterproof covering layer (64) constitutes the dielectric layer, the outer surface of which constitutes the sensing surface, the sensing lines (21,22) can detect the wetness state of the sensing surface through the dielectric layer and by means of electrolytic capacitance, and the detection sensitivity is proportional to the dielectric constant of the dielectric layer and inversely proportional to the thickness of the dielectric layer.

4. A system device for monitoring the wet state of a smart absorbent article (10), comprising a sensor (30) and a smart absorbent article (10) as described in claim 1, wherein the sensor (30) comprises at least two contacts for contacting and electrically connecting the sensing lines (21,22) in the built-in pocket (26), and the sensor (30) also comprises a capacitance detecting device for realizing the wetness detection function of the disposable absorbent article by means of electrolytic capacitance.

5. The system device according to claim 4, wherein the sensor (30) comprises at least three contacts, two of which constitute an insertion detection device, when the sensor (30) is inserted into the built-in pocket (26) and the two contacts contact the same sensing line at the same time, the insertion detection device is triggered, and the sensor (30) enters working state.

6. The system device according to claim 4, wherein the sensor (30) also comprises a wireless transmitter and a wireless receiving device for sending and receiving wetness information of the smart absorbent article (10) by wireless means, and the wireless receiving device comprises an audible and visual alarm device, a smart phone, or a personal computer.

## Patentansprüche

1. Intelligenter Absorptionsartikel (10) auf Grundlage einer kapazitiven Messfolie (40) und einer integrierten Tasche (26), umfassend einen Einwegabsorptionsartikel, eine kapazitive Messfolie (40), eine integrierte Tasche (26) und einen Kurzschlussschutzmechanismus, wobei der Einwegabsorptionsartikel eine Decklage (11), eine Absorptionsschicht (12) und eine Trägerlage (15) umfasst und die Messfolie (40) eine Kontaktoberfläche (61), eine Messoberfläche (62), eine dielektrische Schicht und wenigstens zwei Messleitungen (21, 22) umfasst, wobei die Außenoberfläche der dielektrische Schicht die Messoberfläche ausbildet, die dielektrische Schicht die Messleitungen (21, 22) und die Messoberfläche (62) trennt und isoliert, wenn die Messoberfläche (62) mit einer Feuchtigkeit durchtränkt ist, die Elektrolyt enthält, die Messleitungen (21, 22) die Nässeerfassungsfunktion der Messoberfläche (62) durch die dielektrische Schicht mittels elektrolytischer Kapazität erzielen können, die integrierte Tasche (26) dazu ausgelegt ist, einen Sensor darin einzuführen, die Kontaktoberfläche (61) einen Flächenkontakt und eine elektrische Verbindung zwischen dem Sensor und den Messleitungen (21, 22) ermöglicht und der Kurzschlussschutzmechanismus dazu ausgelegt ist, die Messleitungen (21, 22) vor Feuchtigkeit innerhalb und außerhalb des Einwegabsorptionsartikels zu schützen;
wobei wenigstens ein Teil der Messleitungen (21, 22) in der Kontaktoberfläche (61) angeordnet ist und einen freiliegenden Teil beinhaltet, der Kurzschlussschutzmechanismus eine wasserfeste Deckschicht (64) umfasst, die die Kontaktoberfläche (61) abdeckt, und ein trennbarer Teil zwischen der Kontaktoberfläche (61) und der wasserfesten Deckschicht (64) die integrierte Tasche (26) ausbildet, wobei die Messleitungen (21, 22) die Nässeerfassungsfunktion des Absorptionsartikel durch die dielektrische Schicht mittels elektrolytischer Kapazität erzielen können; und
die kapazitive Messfolie (40) eine Messfolie mit einer integrierten Kopf- und Fußendtasche umfasst, die Messfolie einen Kunststofffolienträger (45) umfasst, die Messleitungen (21, 22) auf einer Oberfläche des Kunststofffolienträgers (45) angeordnet sind, die wasserfeste Deckschicht (64) die Messleitungen (21, 22) abdeckt, die wasserfeste Deckschicht (64) und der Kunststofffolienträger (45) einen Teil der gegenseitigen Verbindung im mittleren Abschnitt beinhalten und so einen Messbereich ausbilden, und einen Teil der Ablösung voneinander am Kopf- und Fußende beinhalten und so die integrierte Tasche (26) ausbilden, wobei die Außenoberfläche des Kunststofffolienträgers (45) oder der wasserfesten Deckschicht (64) in Richtung der Absorptionsschicht (12) gerichtet ist; oder
die kapazitive Messfolie (40) eine Messfolie mit einer durchgängigen integrierten Tasche umfasst, wobei die Messfolie einen Kunststofffolienträger (45) umfasst, die Messleitungen (21, 22) auf einer Oberfläche des Kunststofffolienträgers (45) angeordnet sind und die Kontaktoberfläche (61) ausbilden, die andere Oberfläche des Kunststofffolienträgers (45) die Messoberfläche (62) ausbildet, der Kunststofffolienträger (45) die dielektrische Schicht ausbildet, die wasserfeste Deckschicht (64) die Kontaktoberfläche (61) abdeckt, eine Kante auf beiden Seiten der wasserfesten Deckschicht (64) und des Kunststofffolienträgers (45) einen Teil beinhaltet, an dem sie miteinander verbunden sind, und eine Grenze ausbildet, die das Eindringen von Flüssigkeit darin verhindert, und ferner ein hohler Teil zwischen der wasserfesten Deckschicht (64) und dem Kunststofffolienträger (45) eingeschlossen ist und die durchgängige integrierte Tasche ausbildet.

2. Intelligenter Absorptionsartikel (10) nach Anspruch 1, wobei der Einwegabsorptionsartikel eine Windel, eine Einlage, eine Monatsbinde, ein Mutterschaftstuch oder eine Urinbinde umfasst, wobei die Decklage (11) einen hydrophilen Vliesstoff umfasst, die Absorptionsschicht (12) ein absorbierendes Polymermaterial umfasst, die Trägerlage (15) eine atmungsfähige oder undurchlässige PE-Folie umfasst, die Messleitungen (21, 22) Tintenleitungen, die mit leitfähiger Tinte aufgedruckt sind, Metallleitungen, die durch Gasphasenabscheidung gebildet sind, Aluminiumfolie oder Kupferfolie umfassen, der Kunststofffolienträger eine PE-, PP-, CPP-, BOPP- oder PET-Folie umfasst und die wasserfeste Deckschicht (64) eine Kunststofffolie, ein wasserfestes Papier oder einen wasserabweisenden Vliesstoff umfasst und die einen Elektrolyt enthaltende Flüssigkeit Urin, losen Stuhl, Schweiß oder salzhaltiges Blut umfasst.

3. Kapazitive Messfolie (40) mit einer integrierten Kopf- und Fußendtasche zum Herstellen des intelligenten Absorptionsartikels (10) nach Anspruch 1, umfassend einen Kunststofffolienträger (45), eine wasserfeste Deckschicht (64) und wenigstens zwei Messleitungen (21, 22), wobei die Messleitungen (21, 22) auf einer Oberfläche des Kunststofffolienträgers (45) angeordnet sind, die wasserfeste Deckschicht (64) die Messleitungen (21, 22) abdeckt, die wasserfeste Deckschicht (64) und die Kunststofffolienträger (45) einen Teil der gegenseitigen Verbindung im mittleren Abschnitt beinhalten, um einen geschützten Messbereich zu bilden, um das Eindringen von Flüssigkeit in den Bereich und Verursachen eines Kurzschlusses der Messleitungen (21, 22) zu verhindern, die wasserfeste Deckschicht (64) und der Kunststofffolienträger (45) einen Teil der Ablösung voneinander am Kopf- und Fußende beinhalten und so einen Kontaktbereich ausbilden, wobei der Kontaktbereich an der Vorderkante eine Funktionstasche ausbildet, wobei die Messleitungen (21, 22) in der Funktionstasche freiliegen und eine Kontaktoberfläche (61) für Flächenkontakt und zur elektrischen Verbindung mit den Kontaktstellen des Sensors ausbilden, der in die Tasche eingeführt ist, und der Kontaktbereich an der Hinterkante eine redundante Tasche ausbildet, die Redundanz für die Positionierung eines Schnitts während der Herstellung des intelligenten Absorptionsartikels (10) bereitstellen kann; und
wobei der Kunststofffolienträger (45) oder die wasserfeste Deckschicht (64) die dielektrische Schicht ausbildet, deren Außenoberfläche die Messoberfläche ausbildet, wobei die Messleitungen (21, 22) den Nässezustand der Messoberfläche durch die dielektrische Schicht und mittels elektrolytischer Kapazität erfassen können, wobei die Erfassungsempfindlichkeit proportional zur dielektrischen Konstante der dielektrischen Schicht und umgekehrt proportional zur Dicke der dielektrischen Schicht ist.

4. Systemvorrichtung zum Überwachen des Nässezustands eines intelligenten Absorptionsartikels (10), umfassend einen Sensor (30) und den intelligenten Absorptionsartikel (10) nach Anspruch 1, wobei der Sensor (30) wenigstens zwei Kontakte für den Kontakt und das elektrische Verbinden der Messleitungen (21, 22) in der integrierte Tasche (26) umfasst und der Sensor (30) außerdem eine Kapazitätserfassungsvorrichtung zum Erzielen der Nässeerfassungsfunktion des Einwegabsorptionsartikels mittels elektrolytischer Kapazität umfasst.

5. Systemvorrichtung nach Anspruch 4, wobei der Sensor (30) wenigstens drei Kontakte umfasst, von denen zwei eine Einführerfassungsvorrichtung ausbilden, wenn der Sensor (30) in die integrierte Tasche (26) eingeführt ist, und die zwei Kontakte gleichzeitig mit derselben Messleitung in Kontakt gelangen, woraufhin die Einführerfassungsvorrichtung ausgelöst wird und der Sensor (30) in den Betriebszustand eintritt.

6. Systemvorrichtung nach Anspruch 4, wobei der Sensor (30) außerdem einen Drahtlossender und eine Drahtlosempfangsvorrichtung zum drahtlosen Senden und Empfangen von Nässeinformationen des intelligenten Absorptionsartikels (10) umfasst, wobei die Drahtlosempfangsvorrichtung eine akustische und visuelle Alarmvorrichtung, ein Smartphone oder einen PC umfasst.

## Revendications

1. Article absorbant intelligent (10) basé sur un film de détection capacitif (40) et une poche intégrée (26), comprenant un article absorbant jetable, un film de détection capacitif (40), une poche intégrée (26) et un mécanisme de protection contre les courts-circuits, l'article absorbant jetable comprend une feuille supérieure (11), une couche absorbante (12) et une feuille arrière (15), et le film de détection (40) comprend une surface de contact (61), une surface de détection (62), une couche diélectrique et au moins deux lignes de détection (21, 22), la surface externe de la couche diélectrique constitue la surface de détection, la couche diélectrique sépare et isole les lignes de détection (21, 22) et la surface de détection (62), lorsque la surface de détection (62) est trempée par un liquide contenant de l'électrolyte, les lignes de détection (21, 22) peuvent réaliser la fonction de détection d'humidité de la surface de détection (62) à travers la couche diélectrique au moyen d'un capacitance, la poche intégrée (26) est adaptée pour insérer un capteur, la surface de contact (61) facilite le contact face à face et la connexion électrique entre le capteur et les lignes de détection (21, 22), le mécanisme de protection contre les courts-circuits est adapté pour protéger les lignes de détection (21, 22) de l'humidité à l'intérieur et à l'extérieur de l'article absorbant jetable ;
dans lequel au moins une partie des lignes de détection (21, 22) est située à l'intérieur de la surface de contact (61) et comprend une partie exposée, le mécanisme de protection contre les courts-circuits comprend une couche de revêtement étanche (64) qui recouvre la surface de contact (61), et une partie séparable entre la surface de contact (61) et la couche de revêtement étanche (64) constitue la poche intégrée (26), les lignes de détection (21, 22) peuvent réaliser la fonction de détection d'humidité de l'article absorbant à travers la couche diélectrique au moyen de la capacitance électrolytique ; et
le film de détection capacitif (40) comprend un film de détection avec une poche intégrée de tête et de queue, le film de détection comprend un substrat de film plastique (45), les lignes de détection (21, 22) sont disposées sur une surface du substrat de film plastique (45), la couche de couverture étanche (64) est recouverte sur les lignes de détection (21, 22), la couche de revêtement étanche (64) et le substrat de film plastique (45) comprennent une partie mutuellement liée dans la section centrale et constituent une zone de détection, et comprennent une partie mutuellement séparable aux deux extrémités de la tête et de la queue et constituent la poche intégrée (26), la surface extérieure du substrat de film plastique (45) ou la couche de recouvrement imperméable (64) est orientée vers la couche absorbante (12) ; ou
le film de détection capacitif (40) comprend un film de détection avec une poche intégrée de type traversant, le film de détection comprend un substrat de film plastique (45), les lignes de détection (21, 22) sont disposées sur une surface du substrat de film plastique (45) et constituent la surface de contact (61), l'autre surface du substrat de film plastique (45) constitue la surface de détection (62), le substrat de film plastique (45) constitue la couche diélectrique, la couche de recouvrement imperméable (64) est recouverte sur la surface de contact (61), le bord des deux côtés de la couche de revêtement étanche (64) et le substrat de film plastique (45) comprend une partie collée l'une à l'autre et constitue une limite empêchant la pénétration de liquide, une partie creuse étant en outre incluse entre la couche de revêtement étanche (64) et le substrat de film plastique (45) et constituant la poche intégrée de type traversant.

2. Article absorbant intelligent (10) selon la revendication 1, dans lequel l'article absorbant jetable comprend une couche, un tampon d'insertion, une serviette hygiénique, une serviette maternelle ou un tampon urinaire, la couche supérieure (11) comprend un tissu non tissé hydrophile, la couche absorbante (12) comprend un matériau absorbant polymère, la feuille arrière (15) comprend un film PE respirant ou imperméable, les lignes de détection (21, 22) comprennent des lignes d'encre imprimées avec de l'encre conductrice, des lignes métalliques formées par dépôt physique en phase vapeur, une feuille d'aluminium ou une feuille de cuivre, le substrat de film plastique comprend un film PE, PP, CPP, BOPP ou PET, et la couche de recouvrement imperméable (64) comprend un film plastique, un papier imperméable ou un tissu non tissé hydrofuge, et le liquide contenant de l'électrolyte comprend de l'urine, des selles molles, de la sueur ou du sang contenant du sel.

3. Film de détection capacitif (40) comprenant une poche intégrée de tête et de queue pour la fabrication de l'article absorbant intelligent (10) décrit dans la revendication 1, comprenant un substrat de film plastique (45), une couche de recouvrement imperméable (64) et au moins deux lignes de détection (21, 22), les lignes de détection (21, 22) sont disposées sur une surface du substrat de film plastique (45), la couche de recouvrement étanche (64) recouvre les lignes de détection (21, 22), la couche de recouvrement étanche (64) et le substrat de film plastique (45) comprennent une partie mutuellement liée dans la partie centrale pour former une zone de détection protégée afin d'empêcher la pénétration de liquide dans la zone et de provoquer un court-circuit des lignes de détection (21, 22), la couche de revêtement étanche (64) et le substrat de film plastique (45) comprennent une partie mutuellement séparable aux deux extrémités de la tête et de la queue et constituent une zone de contact, dans lequel la zone de contact située sur le bord avant constitue une poche fonctionnelle, les lignes de détection (21, 22) sont exposées dans la poche fonctionnelle et constituent une surface de contact (61) pour le contact face à face et la connexion électrique avec les points de contact du capteur inséré dans la poche, et la zone de contact située sur le bord d'extrémité arrière constitue une poche redondante, qui peut fournir une redondance d'erreur pour la coupe de positionnement pendant la production de l'article absorbant intelligent (10) ; et
le substrat de film plastique (45) ou la couche de recouvrement imperméable (64) constitue la couche diélectrique dont la surface extérieure constitue la surface de détection, les lignes de détection (21, 22) peuvent détecter l'état d'humidité de la surface de détection à travers la couche diélectrique et au moyen de la capacitance électrolytique, et la sensibilité de détection est proportionnelle à la constante diélectrique de la couche diélectrique et inversement proportionnelle à l'épaisseur de la couche diélectrique.

4. Système de surveillance de l'état humide d'un article absorbant intelligent (10), comprenant un capteur (30) et un article absorbant intelligent (10) selon la revendication 1, dans lequel le capteur (30) comprend au moins deux contacts pour contacter et connecter électriquement les lignes de détection (21, 22) dans la poche intégrée (26), et le capteur (30) comprend également un dispositif de détection de capacité pour réaliser la fonction de détection d'humidité de l'article absorbant jetable au moyen de de capacitance électrolytique.

5. Système selon la revendication 4, dans lequel le capteur (30) comprend au moins trois contacts, dont deux constituent un dispositif de détection d'insertion, lorsque le capteur (30) est inséré dans la poche intégrée (26) et que les deux contacts entrent en contact avec la même ligne de détection en même temps, le dispositif de détection d'insertion se déclenche et le capteur (30) entre en état de fonctionnement.

6. Système selon la revendication 4, dans lequel le capteur (30) comprend également un émetteur sans fil et un dispositif de réception sans fil pour l'envoi et la réception d'informations sur l'humidité de l'article absorbant intelligent (10) par des moyens sans fil, et le dispositif de réception sans fil comprend un dispositif d'alarme sonore et visuelle, un téléphone intelligent ou un ordinateur personnel.
